# EUROPEAN PATENT SPECIFICATION

(11) **EP 1 593 688 B1**
(45) Date of publication and mention of the grant of the patent: **21.11.2007**
(21) Application number: 05103634.1
(22) Date of filing: 02.05.2005
(51) Int. Cl.: C07K 14/59

(54) **Method for partition and inactivation of viral and prion contaminants**
Verfahren zur Abtrennung und Inaktivierung von Virus- und Prionenkontaminationen
Procédé de séparation et d'inactivation de la contamination par des virus et des prions

(30) Priority: 04.05.2004 IT MI20040891
(43) Date of publication of application: 09.11.2005
(73) Proprietor: Ibsa Institut Biochimique S.A., 6903 Massagno (CH)
(72) Inventor: Dattilo, Maurizio, 80124 Napoli (IT)
(74) Representative: Gerli, Paolo

(56) References cited:
- EP-A- 0 530 173
- WO-A-03/100080
- PATENT ABSTRACTS OF JAPAN vol. 1999, no. 06, 31 March 1999 (1999-03-31) & JP 07 187935 A (CHEMOSAN GMBH ERZEUGUNG PHARMAZEUT GRUNDSTOFF; VELITAS KK), 25 July 1995 (1995-07-25)
- REICHL H ET AL.: "Prion transmission in blood and urine: what are the implications for recombinant and urinary-derived gonadotrophins?" HUMAN REPRODUCTION, vol. 17, no. 10, 2002, pages 2501-2508, XP002344561
- KYVSGAARD NC ET AL.: "Effect of Two Virus Inacivation Methods: Electron Beam Irradiation and Binary Ethylenimine Treatment on Determination of Reproductive Hormones in Equine Plasma" ACTA VETERINARIA SCANDINAVICA, vol. 38, no. 3, 1997, pages 225-233, XP008052360 ISSN: 0044-605X

## Description

### FIELD OF THE INVENTION

The present invention relates to the field of decontamination of biological materials. The invention describes a new process for viral and/or prion decontamination, based on the principles of partition and inactivation.

### STATE OF THE ART

It is well known that viral or prion contamination represents a major problem for disinfection of biological materials. This problem occurs particularly, but not exclusively, in down-stream industrial processes for purification of target molecules from blood, urine or culture media. In fact, the susceptibility of target molecules to chemical and non-chemical treatments does not generally allow the use of harsh disinfection procedures without damaging the target molecule itself or, at least, without reducing to very low levels the final yield of the process. In particular, it is well known that small *non-enveloped* viruses and infectious prion proteins represent a very difficult problem to solve.

In the case of viral contaminants, several procedures are available that can contribute to the safety of purification processes. These procedures are typically differentiated in inactivation or partition process. Examples of viral inactivation processes are heat treatments (e.g. pasteurization, lyophilization/dry heat), use of solvents acting as detergents, and high or low pH treatment. Examples of viral partition processes are precipitation processes (e.g. in ethanol, polyethylene glycol), chromatography (ionic exchange, affinity, hydrophobic interaction, reverse phase) and nanofiltration.

EP-A- 0 530 173 shows the treatment of a biological solution wherein urea is added to said solution, obtaining a 8M urea containing composition; the urea can then be removed by chromatography or diafiltration.

Appropriate validation studies must be performed in order to prove the decontamination effectiveness of a purification procedure. Such studies involve the addition to the material, at the beginning of the procedure, of a known amount of infectious agent (so-called "*spiking*") and detection of the presence of residual amounts of the same agent at the end of the procedure. The amount of added infectious agent (*spiked*) is measured as base ten log and the purifying effectiveness of the procedure is expressed as number of Log reduction for the pathogen. Ideally, for regulatory purposes, a purification procedure should show a total viral decontamination efficacy of at least 12-15 Logs. Said efficacy (total Log reduction) should be based on at least three different purification steps that rely on different principles. However, in several purification processes, it is difficult to obtain such result due to the limited applicability of the above procedures to an industrial scale or to their harshness toward the target molecule.

It is even more difficult to eliminate prion contamination, which is much less documented and recently raised major biosafety concerns. In fact, due to their chemical nature and small size, prions escape many types of partition processes that are effective on viruses, and their inactivation is also very difficult to achieve. Exposure to highly alkaline solutions and urea-induced denaturation are among the processes known to induce substantial prion inactivation. However, such procedures often turn out to be harmful to target molecules, thus having little applicability for industrial use.

Therefore, there is a significant need for processes that can be easily implemented in the industry. Said processes should be able to effectively decontaminate solutions containing viruses and/or prions, while maintaining the activity of valuable target molecules.

### SUMMARY OF THE INVENTION

We have now discovered a novel method for partition and inactivating viral and prion contaminants that is based on ultracentrifugation of the test material (starting solution) in a centrifuge tube, where a high-molarity urea solution is layered at the bottom of the centrifuge tube, with the test material placed above this layer. In a preferred embodiment, a third solution composed of polysaccharides is layered as cushion at the interface between the two solutions. Following ultracentrifugation at given g (gravitational acceleration) values and time, contaminating particles are sedimented at the bottom of the tube, within the urea phase, where inactivation takes place. The test material free of contaminating agents can be then selectively recovered as supernatant, or possibly mixed with the other two layers, according to necessities.

### DETAILED DESCRIPTION OF THE INVENTION

The subject of the present invention is a process for partition and inactivating prions and/or viruses that contaminate a starting solution. The process is characterized by the use of ultracentrifugation as partition procedure and of high-molarity urea, layered at the bottom of the centrifuge tube, as inactivating component.

The term "partition" refers to separation of the starting biological material (e.g. the solution) into two sub-fractions in which two components that were mixed together in the starting material are selectively separated (in our case, the target protein on one side and the viruses/prions on the other). The term "inactivation" refers to reduction or elimination of the infectious ability of viruses and prions.

In outline, the procedure involves the preparation in a centrifuge tube of a liquid system composed of layers, with the high-molarity urea solution as bottom layer and the starting solution as top layer. Upon centrifugation, the contaminating agents leave the starting solution and sediment in the lower urea phase, where inactivation takes place. The starting solution, decontaminated by this means, can be then separated and used as needed.

A characteristic feature of this process is that it makes possible to achieve simultaneously both partition and inactivation of viral and/or prion contaminants. In fact, commonly used purification steps can produce either partition or inactivation, but cannot produce both results. Partition alone does not ensure complete disinfection because very small amounts of contaminating agent can still cause disease. Inactivation alone is not sufficient because it is generally incomplete and because of the persistence of immunogenic epitopes of the contaminating agent that can be harmful. The new method proposed here provides both partition and inactivation, by means of an easily applicable procedure. However, concurrent partition and inactivation represents only the most successful scenario, while the invention is also valuable in the case of materials/contaminants that can be partitioned only.

Another important aspect is the minimal contact or the absence of any contact between molecules of industrial interest possibly contained in the starting solution (herein defined as "target molecules") and the urea solution with high denaturing power. This feature allows a harsh treatment to be carried out against contaminating agents without damaging the activity of target molecules present in the starting solution.

The starting solution undergoing decontamination is typically, but not exclusively, a biological fluid of human origin, such as blood, serum, urine, or any other physiological fluid. This solution may contain target molecules that must be preserved during the decontamination process. Target molecules can be pharmacologically active substances, for example proteins (enzymes, cytokines, hormones). An example of target hormone is FSH, contained in high amount in post-menopausal urine. The present procedure can be used to remove from urine, and from downstream purification products, any virus/prion originating from the donor and thus recover FSH or other target molecules in a pure, non-contaminated form.

This process is not limited to medical applications or purification of natural products. The starting solution can be represented also by intermediates or end products of industrial purification processes of several substrates or waste from procedures of chemical synthesis or fermentative processes, etc. Moreover, the procedure is not limited to disinfection of samples that are originally in liquid form, but includes the treatment of any solid material, provided that this can be properly solubilized in a liquid, typically in an aqueous carrier. The resulting solution is used as starting solution in the process described herein; the solid material can be subsequently recovered from the decontaminated solution upon removal of the solvent by means of techniques well known in the art. Generally, any liquid sample containing viral or prion loads can be effectively decontaminated by means of the present invention.

The high-molarity urea solution is a solution containing a urea concentration substantially ranging from 4M to 12 M, preferably from 6M to 10 M, more preferably being 8M. Said solutions are characterized in that they possess a high inactivating power against viruses and prions. At the same time, due to their high molarity, these solutions have a higher density compared to the starting solution, such that they form a heavier liquid phase, separated from the latter, that can be layered at the bottom of the test-tube. The present invention also refers to the use of decontaminating substances other than urea, that can provide density values and inactivation power equivalent to that of the aforesaid urea solutions.

In a preferred embodiment, the present process includes the use of a third solution ("cushion solution") with intermediate density between the starting solution and the urea solution, that is positioned as an intermediate liquid phase between the two solutions. Said cushion solution with intermediate density contains inert substances such as saccharides, salts, physiological solution components, buffers, etc., and is preferably characterized by a neutral or physiological pH. In the essence, the role of solutes in the intermediate solution is to regulate density, providing intermediate density values. Useful reference density values of the cushion solution include, without being limited to, a range between 1.0 and 1.32 g/ml. The term "inert" refers to those solutes that are compatible with the starting solution, i.e. do not exert undesired effects (denaturation, inactivation, precipitation, etc.) on the valuable substances present in such solution. Said inert saccharidic solutes can be monosaccharides, disaccharides, oligosaccharides, polysaccharides. A particularly preferred solute is sucrose that can be used, for example, as solution in 10 mM sodium phosphate, pH 7.4, with a density ranging from 1.0 to 1.32 g/ml. Said solution does not exert undesired effects on the proteins contained in the starting solution and does not prevent particle sedimentation. Moreover, sucrose has the advantage of being readily watersoluble. Finally, it is inexpensive and easily available, thus it is suitable for large-scale processes. A 10% w/v sucrose concentration was used in the examples; however, alternative sucrose concentrations can be used for different test materials.

In presence of the cushion solution, the liquid system undergoing centrifugation is composed of three layers, with the urea solution at the bottom, the cushion solution as intermediate layer, and the starting solution as top layer. After centrifugation, the upper layer containing the starting solution is recovered together with part of the intermediate ("upper sample"), while the remainder of the intermediate layer together with the urea layer form the "lower sample". In a preferred embodiment of the present process, the lower sample consists of the urea layer and of the lower two-thirds of the intermediate (e.g. sucrose) layer; conversely, the upper sample consists of the starting solution and of the upper third of the intermediate (e.g. sucrose) layer.

The use of a cushion solution provides several advantages. The cushion solution, by forming a barrier between the starting solution and the urea phase, eliminates the risk of partially inactivating target molecules present at the interface with the urea solution (which possesses an elevated denaturing activity). At the end of the process, the cushion solution reduces the risk of new contamination possibly caused by reflux or mobilization of the pellet formed at the bottom of the tube. Finally, at the end of the process, the cushion solution makes possible to recover easily and quantitatively the starting solution by aspiration, preventing the collection of part of the urea phase. Aspiration of part of the intermediate layer together with the starting solution does not lead to toxicological problems, nor inactivates valuable components of the solution, and allows recovery of fractions of target molecules that may have diffused in the underlying sucrose layer during the processing time.

Common ultracentrifuges are used for the present process. No particular features of the ultracentrifuge apparatus or of centrifuge tubes are required. Centrifugation conditions generally range from 50.000 to 500.000 g (gravitational accelerations) for a time length between 0.5 and 10 hours, more preferably between 1 and 6 hours, at a temperature ranging between 2 and 8 °C. Compared to the final volume of the liquid system, the starting solution preferably consists of approximately 70% v/v, the urea solution consists of approximately 15% v/v, and the cushion solution consists of approximately 15% v/v.

The process claimed herein includes ultracentrifugation at any scale, from laboratory to industrial scale. The process according to the present invention also includes the use of apparatuses that can be characterized by different structures, but are functionally equivalent to the ultracentrifuge and centrifuge tubes.

The process described herein is widely applicable to partition and inactivation of any viral and prion component. Viruses that can be partitioned/inactivated according to the invention include, without being limited to, DNA viruses, RNA viruses, retroviruses, etc. An example of retrovirus is the Human immunodeficiency virus (HIV); examples of enveloped RNA virus are the Bovine viral diarrhoea virus (BVDV) or the hepatitis C virus (HCV); an example of non-enveloped DNA virus is the Minute virus of mice (MVM); an example of non-enveloped RNA virus is the Hepatitis A virus (HAV). An example of prion is the hamster scrapie prion protein (PrP^{Sc}) 236K.

The invention is illustrated herein by the following examples, without being limited thereto.

### EXPERIMENTAL PART

### Materials and methods

### 1. Choice of the starting solution

The starting solution was selected to be representative of the end product of a purification process from a biological sample carrying a potential viral or prion contamination. Furthermore, the starting solution should have contained suitable amounts of the target molecule.

Therefore we selected the end product of a purification process from postmenopausal urine that is strongly enriched in human follicle - stimulating hormone (FSH). In fact, FSH is a complex heterodimeric glycoprotein, whose structure and function can be damaged easily by harsh downstream treatments, thus it was selected as the most representative example.

More particularly, we used a purification batch from postmenopausal urine, containing a protein concentration of 1 mg/ml and an estimated FSH activity of 6578 IU per mg protein, as measured by an *in vivo* biological assay in rat, and an estimated FSH activity of 5893 IU per mg protein, as measured by EIA. The biological assay in rat was carried out as described in the European Pharmacopoeia.

To test the decontaminating efficacy of the procedure, known amounts of infectious agents, that were appropriately selected for the test, were added to this material, in a process known as *"spiking".*

### 2. Preparation of the cushion solution

We have used as cushion solution a solution of sucrose in 10 mM sodium phosphate, pH 7.4. In the following examples, the sucrose solution was 10% w/v.

### 3. 3 Preparation of the urea solution

We prepared and used a 8M urea solution.

### 4. 4 Preparation of the centrifuge tube

7.0 ml of starting solution, to which known amounts of infectious agent have been added (spiking), were placed in a centrifuge tube having a capacity of 12.0 ml. The cushion solution (1.5 ml) was then layered at the bottom of the tube, using a glass capillary connected to a peristaltic pump, that was completely immersed in the above solution. Finally, 1.5 ml of 8M urea solution were layered at the bottom of the tube by the same procedure.

### 5. Centrifugation conditions

In the following examples, we used a fixed acceleration of 250.000 x g for 4 hours at 4 °C. It is assumed that all infectious particles tested are fully pelleted at this acceleration/time. Therefore, these conditions were used to test the stability of the phases during centrifugation and their recovery. As required, phases remained separated to the end of all experiments.

We used a Sorvall TH 641 swinging bucket rotor with 12.0 ml polyethylene tubes.

### 6. Process of inactivation and recovery of the test sample

At the end of the cycle, we collected by aspiration the upper 7.5 ml corresponding to the phase containing the test sample, and 0.5 ml of the sucrose phase (upper sample). The pellet was resuspended in the remaining 2.5 ml pipetting up and down with a 2.0 ml plastic pipette, and was collected separately (lower sample).

For cell infection assays, because of the cytotoxicity of high molar urea, the lower sample was further treated as follows. The 2.5 ml sample was diluited 1:10 with tissue culture medium to a final volume of 25 ml. It was then ultracentrifuged for 20 minutes at 500.000 x g. At the end, the pellet was resuspended in 2.5 ml of tissue culture medium.

### 7. Assessment of the activity of the target molecule before and after the process

To ascertain the preservation of the target molecule after centrifugation under the above conditions, a non-spiked tube (i.e. without addition of infectious agents that would have made FSH tests impossible or unreliable), containing a FSH control sample, was prepared as described for the spiked tube. After centrifugation, the upper and lower samples from the non-spiked test-tube were tested for FSH activity by an in vivo test in rat and by EIA. It was found that FSH activity did not change in the upper sample (97% and 104% for in vivo test and EIA test, respectively), while remained undetectable in the lower sample.

### 8. 8 Selection of test viruses and prion

The following viruses were selected and tested:
Human immunodeficiency virus type 1 (HIV-1), was selected as an example of retrovirus.
Bovine viral diarrhoea virus (BVDV), a model virus for human hepatitis C virus, was selected as an example of enveloped RNA virus.
Minute virus of mice (MVM), a model virus for parvovirus B19, was selected as an example of non-enveloped DNA virus.
Hepatitis A virus (HAV), was selected as an example of non-enveloped RNA virus.
Moreover, the hamster scrapie prion protein (PrPSc) 236K was selected as representative of pathogenic prion proteins.

### Example 1: partition/inactivation of HIV-1

We used a HIV-1 virus stock, Lai strain, with a titer of 2.63*10⁸ TCID₅₀/ml (tissue culture infectious dose 50% per millilitre), as determined by a quantitative assay on MT4 cells. An aliquot of 0.4 ml of the viral stock was added to 19.6 ml of FSH solution, making up a total volume of 20 ml and yielding a theoretical infectious titer of 5.26*10⁶. The effective viral titer of the spiked solution was determined by a quantitative assay on MT4 cells and was found to be 4.67*10⁶ TCID₅₀/ml.

An aliquot of 7.0 ml of the spiked solution was placed in a centrifuge tube and underlayered with 1.5 ml of 10% w/v sucrose in 10 mM sodium phosphate, pH 7.4, and with 1.5 ml of 8M urea, as previously described (test tube).

A second tube was prepared in the same way and used as non-centrifuged control (control tube).

The test tube was centrifuged at 250.000 x g for 4 hours at 4 °C and then the upper and lower samples were collected and treated according to previously described conditions and procedures. Likewise, upper and lower samples were collected from the control tube.

Upper and lower samples collected from both test and control tubes were assayed for viral infectivity.

Results of quantative assay on MT4 cells: after centrifugation, the viral titer found in the test tube was below the detection limit in both upper and lower samples, indicating that, in the lower sample, complete partition and inactivation was achieved. It was calculated that the resulting Log reduction for partition and inactivation was 4.98 Logs and 5.46 Logs, respectively.

The control tube maintained the entire viral activity within the upper sample, while only little infectivity was detected in the lower sample, possibly due to minimal diffusion of the virus in the sucrose phase during the 4-hour incubation period.

Table 1 shows a summary of the results obtained in example 1.

### Example 2, partition/inactivation of BVDV

We used a BVDV stock, strain NADL (ATCC VR-534), with a titer of 1.53*10⁷ plaque forming units (PFU) per ml, as determined by agarose plaque assay on MBDK cells. An aliquot of 0.2 ml of the viral stock was added to 19.8 ml of FSH solution, making up a total volume of 20 ml and yielding a theoretical infectious titer of 1.53*10⁵. The effective viral titer of the spiked solution was determined by agarose plaque assay on MBDK cells and was found to be 2.3*10⁵ PFU/ml.

An aliquot of 7.0 ml of the spiked solution was placed in a centrifuge tube and underlayered with 1.5 ml of 10% w/v sucrose in 10 mM sodium phosphate, pH 7.4, and with 1.5 ml of 8M urea, as previously described (test tube).

A second tube was prepared in the same way and used as non-centrifuged control (control tube).

The test tube was centrifuged at 250.000 x g for 4 hours at 4 °C and then the upper and lower samples were collected and treated according to previously described conditions and procedures. Likewise, upper and lower samples were collected from the control tube.

Upper and lower samples collected from both test and control tubes were assayed for viral infectivity.

Results of the agarose plaque assay on MBDK cells: after centrifugation, the viral titer found in the upper sample of the test tube was below the detection limit, indicating that, in the lower sample, complete partition and inactivation was achieved. Total infectivity recovered in the lower sample of the test tube was considerably below the amount that was applied, indicating that significant inactivation occurred. It was calculated that the resulting Log reduction for partition and inactivation was 5.08 Logs and 3.18 Logs, respectively.

The control tube maintained the entire viral activity within the upper sample, while only little infectivity was detected in the lower sample, possibly due to minimal diffusion of the virus in the sucrose phase during the 4-hour incubation period.

Table 2 shows a summary of the results obtained in example 2.

### Example 3, partition/inactivation of MVM

We used a stock of MVM, prototype strain, with a titer of 3.27*10⁹ plaque forming units (PFU) per ml, as determined by agarose plaque assay on A9 cells. A 19.8 ml aliquot of FSH solution was spiked with 0.2 ml of viral stock, making up a total volume of 20 ml and yielding a theoretical infectious titer of 3.27*10⁷. The effective viral titer of the spiked solution was determined by agarose plaque assay on A9 cells and was found to be 5.75*10⁷ PFU/ml.

An aliquot of 7.0 ml of the spiked solution was placed in a centrifuge tube and underlayered with 1.5 ml of 10% w/v sucrose in 10 mM sodium phosphate, pH 7.4, and with 1.5 ml of 8M urea, as previously described (test tube).

A second tube was prepared in the same way and and used as non-centrifuged control (control tube).

The test tube was centrifuged at 250.000 x g for 4 hours at 4 °C and then the upper and lower samples were collected and treated according to previously described conditions and procedures. Likewise, upper and lower samples were collected from the control tube.

Upper and lower samples collected from both test and control tubes were assayed for viral infectivity.

Results of the agarose plaque assay on A9 cells: after centrifugation, the viral titer found in the upper sample of the test tube was below the detection limit, indicating that, in the lower sample, complete partition and inactivation was achieved. Total infectivity recovered in the lower sample of the test tube was considerably below the amount that was applied, indicating a high degree of inactivation. It was calculated that the resulting Log reduction for partition and inactivation was 7.05 Logs and 6.02 Logs, respectively.

The control tube maintained the entire viral activity within the upper sample, while only little infectivity was found in the lower sample, possibly due to minimal diffusion of the virus in the sucrose phase during the 4-hour incubation period.

Table 3 shows a summary of the results obtained in example 3.

### Example 4, partition/inactivation of HAV

We used a stock of HAV, HM strain, with a titer of 2.7*10⁹ plaque forming units (PFU) per ml, determined by agarose plaque assay on Frhk-4 cells.

A 19.8 ml aliquot of FSH solution was spiked with 0.2 ml of viral stock, making up a total volume of 20 ml and yielding a theoretical infectious titer of 2.70*10⁷.

The effective viral titer of the spiked solution was determined by agarose plaque assay on Frhk-4 cells and was found to be 3.82*10⁷ PFU/ml.

An aliquot of 7.0 ml of the spiked solution was placed in a centrifuge tube and underlayered with 1.5 ml of 10% w/v sucrose in 10 mM sodium phosphate, pH 7.4, and with 1.5 ml of 8M urea, as previously described (test tube).

A second tube was prepared in the same way and and used as non-centrifuged control (control tube).

The test tube was centrifuged at 250.000 x g for 4 hours at 4 °C and then the upper and lower samples were collected and treated according to previously described conditions and procedures. Likewise, upper and lower samples were collected from the control tube.

Upper and lower samples collected from both test and control tubes were assayed for viral infectivity.

Results of the agarose plaque assay on Frhk-4 cells: after centrifugation, the viral titer found in the upper sample of the test tube was below the detection limit, indicating that, in the lower sample, complete partition and inactivation was achieved. Total infectivity recovered in the lower samples indicates that inactivation occurred, although the extent of inactivation was markedly lower than in the previous examples. It was calculated that the resulting Log reduction for partition and inactivation was 6.88 Logs and 1.49 Logs, respectively.

The control tube maintained the entire viral activity within the upper sample, while only little infectivity was found in the lower sample, possibly due to minimal diffusion of the virus in the sucrose phase during the 4-hour incubation period.

Table 4 shows a summary of the results obtained in example 4.

### Example 5, partition/inactivation of PrP^{Sc}

A 10% scrapie brain homogenate (SBH) was prepared using brains from hamsters infected with the hamster-adapted agent 236K and treated as described by Lee D.C. et al. (Journal of Virological Methodos 2000; 84: 77-89). A 20 ml test solution was spiked with SBH to 1%.

A reference dilution series was prepared by diluting the spiked solution in BSA in increments of 0.5 log.

A 7.0 ml aliquot of the spiked solution was placed in a centrifuge tube and underlayered with 1.5 ml of 10% w/v sucrose in 10 mM sodium phosphate, pH 7.4, and with 1.5 ml of 8M urea, as previously described (test tube).

A second tube was prepared in the same way and used as non-centrifuged control (control tube). The test tube was centrifuged at 250.000 x g for 4 hours at 4 °C and then the upper and lower samples were collected and treated according to previously described conditions and procedures. Likewise, upper and lower samples were collected from the control tube.

The upper and lower samples from both test and control tubes were assayed by Western Blotting (WB) for the presence of PrP^{Sc}, according to Lee D.C. et al. (Journal of Virological Methodos 2000; 84: 77-89).

Results of Western Blotting assay: the WB assay on reference dilutions yielded a visible PrP band in the dilution range between 0 and 4.5 log.

Both upper and lower samples of the test tube were negative by WB at any dilution tested. The upper sample of the control tube was positive by WB in the entire range of dilutions tested. The lower sample of the control tube was found positive by WB up to a dilution of 1.0 Log, possibly due to diffusion of PrP^{Sc} in the sucrose phase during the 4-hour incubation period.

In short, complete partition was observed to the detection limit of the method, corresponding to a reduction of 4.5 Log of the infectious load. Likewise, since no WB reactivity was detected even in the lower sample of the test tube, complete inactivation to the detection limit also occurred, corresponding to 4.5 log reduction of the infectious load.

**Table 1 - Results from example 1, HIV-1**

| Sample | | Volume | Quantitative assay on MT4 cells | | | |
|---|---|---|---|---|---|---|
| | | | TCID₅₀/ml | Total TCID₅₀ | Total Log | Log reduction |
| A | Starting sample | 7 ml | 4.67*10⁶ | 3.27*10⁷ | 7.51 | |
| B | Test tube upper sample | 7.5 ml | ≤ 45 | ≤ 3.37*10² | 2.53 | 4.98 (Log A - Log B) |
| C | Test tube lower sample | 2.5 ml | ≤45 | 1.12*10² | 2.05 | 5.46 (Log A - Log C) |
| D | Control tube upper sample | 7.5 ml | 4.21*10⁶ | 2.95*10⁷ | 7.47 | 0.04 A - Log D) (Log A - Log D) |
| E | Control tube lower sample | 2.5 ml | 3.41*10³ | 8.53*10³ | 3.93 | |

**Table 2- Results from example 2, BVDV**

| Sample | | Volume | Agarose plaque assay on MDBK cells | | | |
|---|---|---|---|---|---|---|
| | | | PFU/ml | Total PFU | Total Log | Log reduction |
| A | Starting sample | 7 ml | 2.3*10⁵ | 1.61*10⁶ | 6.21 | |
| B | Test tube upper sample | 7.5 ml | ≤ 1.8 | ≤ 13.5 | 1.13 | 5.08 (Log A - Log B) |
| C | Test tube lower sample | 2.5 ml | 4.27*10² | 1.06*10³ | 3.03 | 3.18 (Log A - Log C) |
| D | Control tube upper sample | 7.5 ml | 1.75*10⁵ | 1.31*10⁶ | 6.12 | 0.09 (Log A - Log D) |
| E | Control tube lower sample | 2.5 ml | 7.42*10³ | 1.85*10⁴ | 4.27 | |

**Table 3 - Results from example 3, MVM**

| Sample | | Volume | Agarose plaque assay on A9 cells | | | |
|---|---|---|---|---|---|---|
| | | | PFU/ml | Total PFU | Total Log | Log reduction |
| A | Starting sample | 7 ml | 5.75*10⁷ | 4.02*10⁸ | 8.6 | |
| B | Test tube upper sample | 7.5 ml | ≤ 4.8 | 36 | 1.55 | 7.05 (Log A - Log B) |
| C | Test tube lower sample | 2.5 ml | 1.53*10² | 3.83*10² | 2.58 | 6.02 (Log A - Log C) |
| D | Control tube upper sample | 7.5 ml | 4.81*10⁷ | 3.6*10⁸ | 8.56 | 0.04 (Log A - Log D) |
| E | Control tube lower sample | 2.5 ml | 1.07*10² | 2.68*10² | 2.43 | |

**Table 4 - Results from example 4, HAV**

| Sample | | Volume | Agarose plaque assay on FrhK-4 cells | | | |
|---|---|---|---|---|---|---|
| | | | PFU/ml | Total PFU | Total Log | Log reduction |
| A | Starting sample | 7 ml | 3.82*10⁷ | 2.67*10⁸ | 8.43 | |
| B | Test tube upper sample | 7.5 ml | ≤4.8 | ≤ 36 | 1.55 | 6.88 (Log A - Log B) |
| C | Test tube lower sample | 2.5 ml | 3.46*10⁶ | 8.65*10⁶ | 6.94 | 1.49 (Log A - Log C) |
| D | Control tube upper sample | 7.5 ml | 3.74*10⁷ | 2.8*10⁸ | 8.45 | -0.03 (Log A - Log D) |
| E | Control tube lower sample | 2.5 ml | 6.33*10⁵ | 1.58*10⁶ | 6.2 | |

## Claims

1. Process for partition and inactivation of viruses and/or prions contaminating a starting solution, **characterized in that** ultracentrifugation is used as partition procedure, a high-molarity urea solution is used as the inactivating component and is layered at the bottom of the centrifuge tube, with the starting solution placed above this layer.

2. Process according to claim 1, wherein high molarity urea is present at concentrations ranging from 6M to 10M.

3. Process according to claims 1-2, wherein a liquid phase interposed between the starting solution and the high molarity urea solution is also present in the centrifuge tube.

4. Process according to claim 3, wherein the interposed liquid phase is a solution containing one or more substances of saccharidic nature.

5. Process according to claims 3-4, wherein the interposed liquid phase is a solution including sucrose, with pH ranging from 6.5 to 8, and a density ranging from 1.0 to 1.32 g/ml.

6. Process according to claims 1-5, wherein the starting solution is a biological material, an intermediate or an end product of a purification process, or a synthetic solution.

7. Process according to claims 1-6, wherein the starting solution contains one or more substances of industrial interest.

8. Process according to claims 1-7, wherein the starting solution is postmenopausal urine or an intermediate or final purification product of the same, and the substance of industrial interest is human follicle stimulating hormone (FSH).

9. Process according to claims 1-8, wherein the contaminating agents are comprised in the group of retroviruses, enveloped or non-enveloped RNA viruses, enveloped or non-enveloped DNA viruses, and infectious prion proteins.

## Patentansprüche

1. Verfahren zur Abtrennung und Inaktivierung von Viren und/oder Prionen, die eine Ausgangslösung kontaminieren, welches **dadurch gekennzeichnet ist, dass** Ultrazentrifugation für das Abtrennverfahren verwendet wird, wobei eine hochmolekulare Harnstofflösung als inaktivierender Bestandteil verwendet und auf den Boden des Zentrifugenröhrchens geschichtet wird und die Ausgangslösung über diese Schicht gegeben wird.

2. Verfahren gemäß Anspruch 1, wobei hochmolekularer Harnstoff in Konzentrationen im Bereich von 6 M bis 10 M vorliegt.

3. Verfahren gemäß Ansprüchen 1-2, wobei auch eine flüssige Phase, die zwischen der Ausgangslösung und der hochmolekularen Harnstofflösung in dem Zentrifugenröhrchen eingefügt ist, in dem Zentrifugenröhrchen vorliegt.

4. Verfahren gemäß Anspruch 3, wobei die dazwischen eingefügte Phase eine Lösung ist, welche eine oder mehrere von Saccharid abgeleitete Substanz(en) enthält.

5. Verfahren gemäß Ansprüchen 3-4, wobei die dazwischen eingefügte flüssige Phase eine Lösung ist, welche Saccharose enthält, mit einem pH-Wert im Bereich von 6,5 bis 8 und einer Dichte im Bereich von 1,0 bis 1,32 g/ml.

6. Verfahren gemäß Ansprüchen 1-5, wobei die Ausgangslösung ein biologisches Material, ein Zwischenprodukt oder ein Endprodukt eines Reinigungsverfahrens oder eine synthetische Lösung ist.

7. Verfahren gemäß Ansprüchen 1-6, wobei die Ausgangslösung eine Substanz oder mehrere Substanzen von gewerblichem Interesse enthält.

8. Verfahren gemäß Ansprüchen 1-7, wobei die Ausgangslösung Urin von postmenopausalen Frauen, ein Zwischenprodukt oder ein Endreinigungsprodukt des gleichen ist, und es sich bei der Substanz von gewerblichem Interesse um humanes Follikel-stimulierendes Hormon (FSH) handelt.

9. Verfahren gemäß Ansprüchen 1-8, wobei die kontaminierenden Agenzien in der Gruppe enthalten sind, die Retroviren, behüllte oder nicht-behüllte RNA-Viren, behüllte oder nicht-behüllte DNA-Viren und infektiöse Prionproteine umfasst.

## Revendications

1. Procédé de partition et d'inactivation de virus et/ou de prions contaminant une solution de départ, **caractérisé en ce que** l'ultracentrifugation est utilisée comme méthode de partition, une solution d'urée à haute molarité est utilisée comme composant d'inactivation et est déposée en couche au fond du tube de centrifugation, la solution de départ étant placée au-dessus de cette couche.

2. Procédé selon la revendication 1, dans lequel l'urée de haute molarité est présente à des concentrations allant de 6M à 10M.

3. Procédé selon les revendications 1-2, dans lequel une phase liquide interposée entre la solution de départ et la solution d'urée à haute molarité est aussi présente dans le tube de centrifugation.

4. Procédé selon la revendication 3, dans lequel la phase liquide interposée est une solution contenant une ou plusieurs substances de nature saccharidique.

5. Procédé selon les revendications 3-4, dans lequel la phase liquide interposée inclut du sucrose, avec un pH allant de 6,5 à 8, et une densité allant de 1,0 à 1,32 g/ml.

6. Procédé selon les revendications 1-5, dans lequel la solution de départ est une matière biologique, un intermédiaire ou un produit final d'un procédé de purification, ou une solution synthétique.

7. Procédé selon les revendications 1-6, dans lequel la solution de départ contient une ou plusieurs substances d'intérêt industriel.

8. Procédé selon les revendication 1-7, dans lequel la solution de départ est de l'urine postménopausale ou un intermédiaire ou un produit final de purification de ce produit, et la substance d'intérêt industriel est une hormone de stimulation folliculaire humain (FSH).

9. Procédé selon les revendications 1-8, dans lequel les agents contaminants sont compris dans le groupe des rétrovirus, des virus à ARN enveloppés ou non, des virus à ADN enveloppés ou non et des protéines de prions infectieux.
